(19) Europäisches Patentamt
European Patent Office
Office européen des brevets

(11) Publication number: **0 081 892**
**B1**

(12) # EUROPEAN PATENT SPECIFICATION

(45) Date of publication of patent specification: **18.03.87**

(21) Application number: **82201608.5**

(22) Date of filing: **15.12.82**

(51) Int. Cl.[4]: **C 07 D 493/08,**
C 07 D 303/14, C 07 C 35/18,
C 07 C 43/196, C 07 C 149/36,
C 07 C 69/608 // (C07D493/08,
307:00, 307:00)

(54) **Process for the preparation of bicycloheptane derivatives, and novel bicycloheptane derivatives.**

(30) Priority: **16.12.81 US 331095**

(43) Date of publication of application:
**22.06.83 Bulletin 83/25**

(45) Publication of the grant of the patent:
**18.03.87 Bulletin 87/12**

(84) Designated Contracting States:
**AT BE CH DE FR GB IT LI LU NL SE**

(56) References cited:

**JOURNAL OF THE AMERICAN CHEMICAL SOCIETY, Vol. 94, no. 8, 19 April 1972, Columbus, Ohio (US); L.A.SPURLOCK et al.: "The nature of the carbonium ion. IX. The 2-oxy-6-norbornyl cation", pp. 2707-2711**

**TETRAHEDRON, vol. 37, no. 23, 1981, London (GB); R.L.DANHEISER et al.: "Cyclohexenol annulation via the alkoxy-accelerated rearrangement of vinylcyclobutanes", pp. 3943-3950**

**Chemical Abstracts, vol. 91, no. 7, 13 August 1979, Columbus, Ohio (US); H.TAKAHASHI et al.: "Synthesis of 7-oxabicyclo2.2.1r-heptanes from 3-cyclohexenols involving photoinduced hydoiodite reaction", p. 727, abstract 57190v**

**Chemical Abstracts, vol. 66, no. 21, 22 May 1967 Columbus, Ohio (US); N.S.ZEFIROV et al.: "3,6-Endoxocyclohexanes and -cyclohexenes. XXXVII. Reaction of oxidative bisdecarboxylation in 7-oxabicycloheptane series", p. 8837, abstract 94597n**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Payne, George Bernson**
**308 Harrow Court**
**Modesto, CA 95350 (US)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 081 892 B1

**Description**

This invention relates to a process for the preparation of bicycloheptane derivatives, and to certain novel bicycloheptane derivatives.

Our co-pending application published as EP 0081893 A2 describes and claims certain novel compounds useful as herbicides. Certain ethers derived from the *exo* isomers of 2-hydroxy-7-oxabicyclo[2,2,1]heptane derivatives are especially useful; such ethers may be prepared from said hydroxy compounds.

J. Chem. Soc. (c), 1969, pages 716—721, describes a process for the preparation of 2-*endo*-hydroxy-1-methyl-4-isopropyl-7-oxabicyclo[2,2,1]heptane. However, no method is known for the preparation of the corresponding *exo* compound and related compounds.

The present invention therefore relates to a process for the preparation of a 2-*exo*-hydroxy-7-oxabicyclo[2,2,1]heptane, which comprises cyclising the corresponding *cis* 3,4-epoxycyclohexanol by treatment with an acid.

In accordance with the present invention there is provided a process for the preparation of a 2-*exo*-hydroxy-7-oxabicyclo[2,2,1]heptane compound of the general formula I by cyclisation, by treatment with an acid, of a *cis* isomer of a 3,4-epoxy cyclohexanol compound of the general formula II

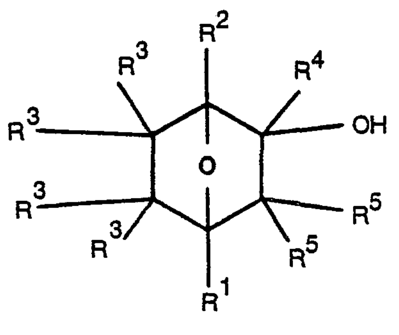

(I)

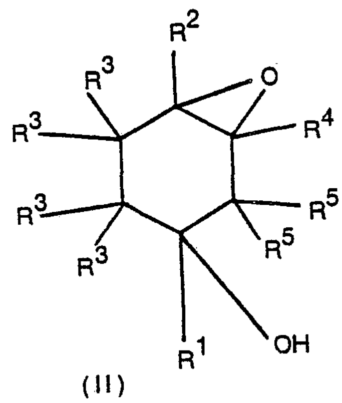

(II)

in which formulae $R^1$ is a hydrogen atom, a $C_{1-10}$ alkyl group optionally substituted by up to 3 fluorine, chlorine and/or bromine atoms or by a hydroxy group, a cyano group, a $C_{1-4}$ alkoxy group, a $C_{1-6}$ alkyl-sulphonyl group, a $C_{6-10}$ arylsulphonyl group, a $C_{7-11}$ aralkylsulphonyl group, an azido group, a $C_{1-6}$ alkoxycarbonyl group, a hydroxycarbonyl group, a phosphoryl group, a phosphoryloxy group, or an amine oxide, carbamoyl or thiocarbamoyl group in which the nitrogen atom is optionally substituted by 1 or 2 $C_{1-4}$ alkyl groups; an aryl or aralkyl group, each containing from 6 to 11 carbon atoms, and 1 to 4 carbons in any alkyl portion, each optionally ring-substituted by one or more fluorine, chlorine and/or bromine atoms or by a $C_{1-2}$ alkyl or alkoxy group optionally substituted by one or more fluorine and/or chlorine atoms; or a group $CO_2R^6$ or $CON(R^6)_2$ in which $R^6$ is a hydrogen atom or a $C_{1-6}$ alkyl group;

$R^2$ is a hydrogen atom or a straight-chain $C_{1-6}$ alkyl group; each $R^3$ is independently a hydrogen, chlorine or bromine atom, or a $C_{1-4}$ alkyl group optionally substituted by up to 3 fluorine, chlorine and/or bromine atoms; or two $R^3$'s when located on adjacent carbon atoms together form an epoxide ring or a carbon-carbon bond;

$R^4$ is a hydrogen atom or a $C_{1-4}$ alkyl group optionally substituted by up to 3 fluorine, chlorine and/or bromine atoms; each $R^5$ is independently a hydrogen atom; a $C_{1-4}$ alkyl group optionally substituted by up to 3 fluorine chlorine and/or bromine atoms; a hydroxy group; or a $C_{1-4}$ alkoxy group.

Preferably $R^1$ is a hydrogen atom; a $C_{1-4}$ alkyl group optionally substituted by up to 3 fluorine, chlorine and/or bromine atoms or by OH, CN, a $C_{1-4}$ alkoxy group, a $C_{1-6}$ alkylsulphonyl group, a phenylsulphonyl group, or a benzylsulphonyl group; or an aryl or aralkyl group each containing from 6 to 10 carbon atoms and 1 or 2 carbon atoms in any alkyl portion, optionally substituted by one or more fluorine, chlorine and/or bromine atoms or by a $C_{1-2}$ alkyl or alkoxy group each optionally substituted by one or more fluorine and/or chlorine atoms.

More preferably, $R^1$ is a hydrogen atom or a $C_{1-3}$ alkyl group optionally substituted by chlorine, for example a methyl, ethyl, n-propyl, isopropyl or 1-chloro-1-methylethyl group.

Preferably $R^2$ is a straight-chain $C_{1-4}$ alkyl group, especially a methyl or ethyl group.

Especially preferred for use in the process of the invention are compounds in which $R^1$ is an isopropyl group and $R^2$ is a methyl group; $R^1$ and $R^2$ are both ethyl groups; or $R^1$ is a 1-chloro-1-methylethyl group and $R^2$ is a methyl group.

Each $R^3$ is preferably independently a hydrogen atom; a chlorine atom; a bromine atom; or a methyl or ethyl group. Most preferably each $R^3$ is a hydrogen atom.

Preferably $R^4$ is a hydrogen atom.

Preferably each $R^5$ is a methyl or ethyl group or, especially, a hydrogen atom.

Most of the compounds of the general formulae I and II are novel. The invention therefore also provides a compound of the general formula I or II *per se*, wherein $R^1$, $R^2$, $R^3$, $R^4$ and $R^5$ have the meanings given above, with the proviso that when $R^2$ represents a methyl group and all of $R^3$, $R^4$ and $R^5$ represent hydrogen atoms, then $R^1$ is other than an isopropyl group.

The cyclization reaction is surprising in its relatively high yield of the *exo*-hydroxy configuration of the resulting 2-hydroxy-7-oxabicyclo[2,2,1]heptane. Many acids will catalyze this reaction, but a relatively strong acid, such as hydrochloric, sulphuric or sulphonic acids, or an acidic ion exchange resin, such as Amberlist (Trade Mark) sulphonic acid, is especially suitable. Preferably, the acid has a $pK_a$ of 1 or less. Most preferably, the acid is an organic sulphonic acid, for example an alkyl, alkaryl or aryl sulphonic acid, such as methanesulphonic, ethanesulphonic, *p*-toluenesulphonic, *m*-nitrobenzenesulphonic, 2,4-dimethylbenzenesulphonic, or benzenesulphonic acid. Of these, *p*-toluenesulphonic acid is preferred.

The amount of acid used is not critical; preferably it is used in amounts from 0.001 to 0.5 mole per mole of *cis*-epoxycyclohexanol, suitably from 0.01 to about 0.1, and especially from 0.02 to 0.04 mole of acid per mole of *cis*-epoxycyclohexanol.

The reaction is preferably carried out in the presence of an inert solvent. Suitable solvents include for example chlorinated hydrocarbons, ethers, hydrocarbons, and ketones. Typical chlorinated hydrocarbons contain from 1 to 4 chlorine atoms in combination with an alkane chain containing from 1 to 4 carbon atoms or a benzene ring, for example carbon tetrachloride, chloroform, dichloromethane, chlorobenzene and 1,2- or 1,3-dichlorobenzene. Preferred ethers are generally those containing from 4 to 6 carbon atoms, for example diethyl ether, methyl tert-butyl ether and diisopropyl ether. Tetrahydrofuran and dioxane are also useful. Suitable alkanes contain for example from 5 to 10 carbon atoms; petroleum fractions rich in alkanes are suitable. Petroleum ether is also suitable. Cyclohexane and methylcyclohexane are examples of useful cycloalkane solvents containing from 6 to 8 carbon atoms. Suitable aromatic hydrocarbon solvents preferably contain from 6 to 10 carbon atoms, for example, benzene, toluene, o-, m-, and p-xylene, the trimethylbenzenes and p-ethyltoluene. Suitable ketones include acetone and methyl ethyl ketone. If an excess of epoxycyclohexanol is present, this may, if desired, serve as solvent so that no additional solvent is necessary. Preferably the reaction is carried out in the substantial absence of water.

The reaction temperature is conveniently in the range of from 0—75°C, in particular from 0°C to 50°C. Suitably, the temperature is from 5°C to 40°C, preferably from 10°C to 30°C.

The resulting 2-*exo*-hydroxy-7-oxabicyclo[2,2,1]heptane derivative may be purified by conventional techniques or converted without isolation into useful ether derivatives.

The *cis* 3,4-epoxycyclohexanol used as starting material in the process according to the invention may be prepared by epoxidation of the corresponding cyclohexenol, using a suitable oxidising agent. Preferred cyclohexenols are those of the general formula

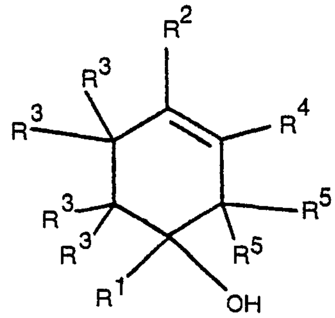

(III)

in which $R^1$, $R^2$, $R^3$, $R^4$ and $R^5$ have the meanings given above.

Any oxidizing agent that will form the epoxyalcohol may be used. Preferred oxidising agents include peroxy acids, hydrogen peroxide and organic peroxides, such as *m*-chloroperbenzoic aid, peracetic acid, perphthalic acid, cumene hydroperoxide, persuccinic acid, pernonanoic acid, an alkyl hydroperoxide such as tert-butyl hydroperoxide, persulphuric acid and hydrogen peroxide.

Preferably, the oxidation is carried out using an alkyl hydroperoxide or hydrogen peroxide and is conducted in the presence of an appropriate transition metal catalyst. Such a catalyst is preferably present in an amount of from about 0.0005 to 0.10 mole of catalyst per mole of peroxide, preferably from about 0.01 to 0.03 mole catalyst per mole of peroxide. Suitable transition metal catalysts are complexes of metals of atomic numbers 22—31, 40—49 and 72—81. Preferably the complex is an organic complex, for example with beta-diketones, o-hydroxybenzaldehydes or o-hydroxybenzophenones and particularly with acetylacetone. Catalysts containing molybdenum or, especially, vanadium, are preferred; for example, vanadium (IV) bis(2,4-pentanedionate) oxide is especially useful.

The oxidizing agent may suitably be used in an amount from 0.5 to 3, preferably in an amount from 1.0 to 1.2 moles per mole of cyclohexenol, most preferably from 1.0—1.1 moles per mole of cyclohexenol.

The treatment with an oxidizing agent is preferably conducted in a solvent, ideally of the type also useful in the subsequent cyclization as described above.

The reaction temperature is conveniently in the range of about −10°C to 75°C, in particular from −10 to 50°C. Generally, the preferred temperature is from −5°C to 40°C, preferably from 10°C to 30°C.

The resulting epoxy-alcohol may be purified by conventional techniques or converted without isolation into the 2-*exo*-hydroxy-7-oxabicyclo[2,2,1]heptane by cyclization.

Especially useful for obtaining a 2-*exo*-hydroxy-7-oxabicyclo[2,2,1]heptane is treatment of the appropriate cyclohexenol with either tert-butyl hydroperoxide and vanadium (IV) bis (2,4-pentanedioate) oxide as catalyst in a solvent, for example methylene chloride, or hydrogen peroxide and the vanadium catalyst in a solvent, for example acetone, followed by treatment of the intermediate epoxide, preferably in *situ*, with a sulphonic acid, particularly *p*-toluenesulphonic acid. In some cases, acid generated during the epoxidation step from a peroxy acid, e.g. *m*-chloroperbenzoic acid, produces the desired cyclised product directly.

The epoxidation and ring closure steps may be carried out successively; alternatively, the 3-cyclohexen-1-ol may be treated conveniently with an oxidizing agent and an acid, to produce cyclisation of the epoxide *in situ*.

The 2-*exo*-hydroxy-7-oxabicyclo[2,2,1]heptanes exhibit geometrical and optical isomerism. From the various optical and geometrical forms of the materials of the invention and various combinations thereof ethers may be prepared which usually have different herbicidal activities. The materials of formula I that have the OH group *exo* (formula Ia below) with respect to the oxygen-containing bridge result in *exo* ethers usually more herbicidally active than the ethers of the *endo* hydroxy form (formula Ib below) or the *exo-endo* mixture.

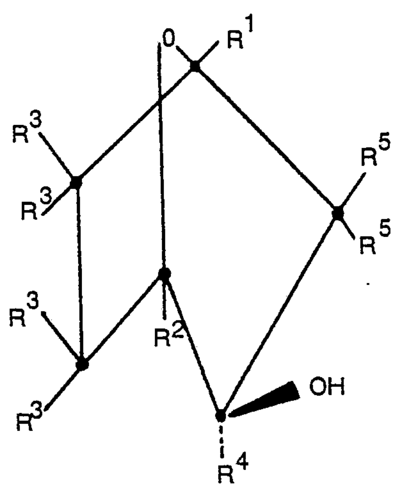

1S—*exo*—form

Formula Ia

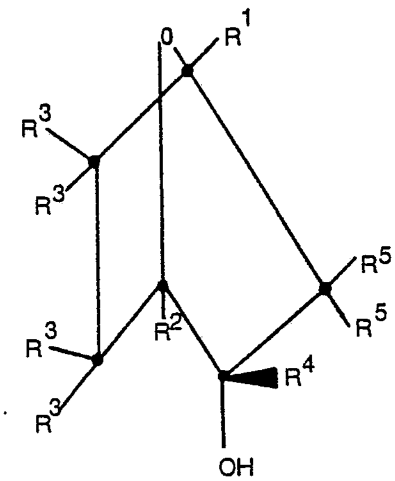

1S—*endo*—form

Formula Ib

When $R^2$ is hydrogen, then the compounds of formula Ia and Ib have the 1S absolute configuration shown above. Such compounds of the invention of Ia that correspond in configuration are preferred. In situations where the *endo* form is desired it can be obtained by oxidation of the 2-*exo*-hydroxy compound to the corresponding ketone following by reduction of the ketone with sodium borohydride.

The compounds of the general formula I are useful intermediates in the preparation of the corresponding novel ethers having herbicidal properties in which the hydrogen of the 2-hydroxy group is replaced by a group $WCQ_2$- in which W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms, optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen; $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; aminocarbonyl, amino and carboxyl in each of which hydrogen may be replaced by $C_{1-4}$ alkyl; and $C_{1-3}$ alkyl which is itself optionally substituted by halogen, hydroxy, $C_{1-4}$ alkylthio, $C_{1-4}$ alkoxy and amino; and both of Q are hydrogen atoms or fluorine atoms. These ethers and their use to control the growth of unwanted plants are disclosed in our copending Application published as EP 0081893A2. Such ethers may be prepared by treating the 2-*exo*-hydroxy-7-oxabicyclo[2,2,1]heptane of formula I with a compound of the formula $WCQ_2L$, in which L is a suitable leaving group, for example a halogen atom, such as bromine, chlorine or iodine or an organic sulphonyloxy group, for example a mesyloxy or tosyloxy group. The

reaction is preferably carried out in the presence of a strong base and an inert diluent, and preferably in the presence of a catalyst. The strong base is suitably an alkali metal hydride, amide, hydroxide or carbonate, including, for example, sodium hydride, sodium amide, sodium hydroxide and potassium carbonate. Inert diluents are suitably organic solvents, such as ethers, sulphoxides, aromatic hydrocarbons and chlorinated hydrocarbons including for example, diethyl ether, tetrahydrofuran, toluene and methylene chloride. Suitable catalysts are organic bases, such as tertiary amines and ammonium compounds, for example triethylamine or benzyltriethylammonium chloride. The reaction is most conveniently carried out under normal pressures and ambient temperatures. Suitable temperatures for the reaction are from about 0° to about 120°C, preferably from 20° to 100°C.

The 3-cyclohexen-1-ols can be synthesized as described below or obtained from natural sources.

(a) Where $R^2$ is methyl and $R^1$ is isopropyl, and the remaining R's are hydrogen, the compound is terpinen-4-ol, which occurs naturally in optically active and racemic forms, or can be prepared by epoxidation of terpinolene, followed by reduction of the epoxide.

(b) Substituted-1-oxaspiro(2,5)oct-5-enes are useful for preparing 3-cyclohexen-1-ols where $R^1$ is substituted by OH, OR, SR, $NR_2$, $N_3$, $P(O)(OR)_2$. Treatment of a 1-oxaspiro(2,5)oct-5-ene with water or an alcohol in the presence of a strong acid affords the corresponding 3-cyclohexen-1-ol substituted in the 1-position by a hydroxymethyl or alkoxymethyl group. Treatment of 1-oxaspiro(2,5)oct-5-ene with thiophenol, or an alkyl, $C_{6-11}$ aryl, or $C_{7-11}$ aralkyl mercaptan, in the presence of a catalyst, such as sodium hydride, and a suitable solvent, produces a thio-substituted 3-cyclohexen-1-ol that can be converted to the corresponding sulphonyl derivative in the course of the oxidation process described above. Where $R^1$ is halo-substituted, the haloalkyl-substituted-3-cyclohexen-1-ol may be prepared by treating a spiro compound as defined above with an ethereal hydrohalogenic acid, e.g. hydrochloric acid. From the (haloalkyl) substituted compounds thus obtained (haloalkyl)-substituted ethers may be made which can then be dehydrochlorinated to yield the corresponding 4-alkenyl-substituted ethers (where $R^1$ is alkenyl) with the use of a base. Compounds where $R^1$ is alkenyl may also be made by rearrangement of the spiro compounds upon treatment with protic or Lewis acids. Where $R^1$ is substituted by an amine oxide group, a spiro compound as defined above may be treated with the appropriate dialkylamine in the presence of a catalyst such as triethylaluminum; the subsequent epoxidation step produces the amine oxide. Where $R^1$ is dialkoxyphosphoryl, the compounds may be prepared by treatment of a 1-oxaspirol-[2,5]oc-5-ene with the appropriate phosphite ester.

(c) Preparation of 3-cyclohexen-1-ols can be effected from suitably substituted phenols by Birch-type reduction: see for example Rodd's Chemistry of Carbon Compounds, Second Edition, *Vol. II, Part B,* pages 1—4 (1968).

(d) Where $R^1$ is $CO_2R^6$, $CON(R^6)_2$, CN, or alkyl, the 3-cyclohexen-1-ols can be prepared starting from suitable Diels-Alder adducts, followed where appropriate by modification of $R^1$.

Examples 1 to 10

These Examples describe the preparation of compounds of the general formula III.

Example 1

1,4-Diethyl-3-cyclohexen-1-ol

To a stirred refluxing mixture of 600 ml of dry diethyl ether and 1600 ml of liquid ammonia was added 136 g of *p*-ethylanisole. After 15 minutes, there was added portionwise, at −35 to 32°C, a 26.4 g quantity of lithium ribbon over 1 hour. After an additional 15 minutes, 193 g of dry ethanol was added dropwise at −35° to −32°C. Stirring was continued until the blue color disappeared, and the ammonia was allowed to evaporate on standing overnight. The residue was poured into 1 liter of ice water and extracted twice with diethyl ether. The combined ether extracts concentrated to a volume of about 300 ml were stirred with 250 ml of water containing 46 g of oxalic acid overnight at ambient temperature. This mixture was diluted with 1 liter of water and extracted twice with diethyl ether. The combined ether extracts were washed with 5% sodium bicarbonate and then with water. After drying, the ether solution was vacuum-concentrated to a residue of 104.4 g of 4-ethyl-3-cyclohexen-1-one; it was 94% pure by GLC analysis. 10.0 g of this product was dissolved in 25 ml of diethyl ether, and added dropwise to a stirred solution of 35 ml of 3.2 M ethereal ethyl magnesium bromide in 75 ml of dry diethyl ether at gentle reflux. After one hour longer at reflux, the mixture was cooled and treated dropwise with 80 ml of water. The aqueous layer was extracted with diethyl ether and the combined ether layers were dried, concentrated, and Claisen distilled to give 7.3 g of the desired product, b.p. 82—86°C (5 mm, 665 Pa).

Example 2 (deleted)

Example 3

(±)-4-Methyl-(1-(1-chloro-1-methylethyl)-3-cyclohexen-1-ol

To a stirred solution of 15.2 g of 2,2,6-trimethyl-1-oxaspiro(2,5)oct-5-ene in 200 ml of diethyl ether held at −10°C was added dropwise 32 ml of 3.8 N ethereal hydrochloric acid. After one hour at 0—5°C, the mixture was washed with three 50 ml portions of water, dried and distilled to give 14.5 g of the desired product, b.p. 70—75°C (0.4 mm, 53 Pa).

Example 4

(±)-1-Hydroxy-alpha,alpha,4-trimethyl-3-cyclohexen-1-acetonitrile

To a stirred mixture of 7.0 g of zinc dust, 0.45 g of mercuric chloride and 4 ml of tetrahydrofuran, was added dropwise over 45 minutes at 20—25°C a mixture of 9.7 g of 4-methyl-3-cyclohexen-1-one, 13.7 g of alpha-bromoisobutyronitrile and 25 ml of tetrahydrofuran. After an additional hour at 25°C, the reaction mixture was cooled to 5—10°C and treated dropwise with 50 ml of cold 10% sulphuric acid. To this was added 100 ml of methylene chloride, and the mixture was filtered. The filtrate was diluted with 100 ml of water and extracted twice with 100 ml portions of methylene chloride. The combined methylene chloride extracts were washed with aqueous sodium bicarbonate, dried and Claisen distilled to give 11.3 g of the desired product. b.p. 92—102°C (0.25 mm, 33 Pa).

Example 5

(±)-1-(1-hydroxy-1-methylethyl)-4-methyl-3-cyclohexen-1-ol

A mixture of 26.0 g of 2,2,6-trimethyl-1-oxaspiro(2,5)oct-5-ene and 250 ml of 1% sulphuric acid was stirred magnetically for 20 hours, then extracted with four 100 ml portions of methylene chloride. The combined methylene chloride extracts were washed, dried, concentrated and Claisen distilled to give 22.4 g of the desired product, b.p. 78—81°C (0.15 mm, 20 Pa).

Example 6

(±)-4-Methyl-1-(1-methoxy-1-methylethyl)-3-cyclohexen-1-ol

To a stirred solution of 0.8 g of *p*-toluenesulphonic acid in 125 ml of methanol held at 3—5°C was added dropwise over 0.5 hour a solution of 15.2 g of 2,2,6-trimethyl-1-oxaspiro(2,5)oct-5-ene in 25 ml of methanol. After an additional 2 hours at 5°C and 2 hours at 5—20°C, the mixture was treated with 2 ml of 15% sodium hydroxide and concentrated at a water pump at below 60°C. The residue was dissolved in methylene chloride, washed, dried and Claisen distilled to give 15.6 g of the desired product, b.p. 70°C (0.2 mm, 27 Pa).

Example 7

2,2,4-trimethyl-3-cyclohexen-1-ol

A mixture of 352 g of *p*-methyldihydroanisole (85% purity), 1350 ml of diethyl ether, 38 g of oxalic acid and 900 ml of water was stirred mechanically for 21 hours at 25°C. The aqueous layer was separated and extracted twice with diethyl ether. The combined ether solutions were washed with sodium bicarbonate, dried, concentrated and Claisen distilled to give 250 g of 4-methyl-3-cyclohexen-1-one, b.p. 63—65°C (13 mm, 1730 Pa).

To a 500 ml 3-neck, round-bottom flask were charged 37.5 g of this product, 100 ml of diethyl ether, 89.5 g of methyl iodide and 0.3 g of methyltrioctylammonium chloride. This mixture was stirred mechanically at ambient temperature and treated with 30 g of granular sodium hydroxide. After 20 minutes at gentle reflux, heat was applied to maintain the reflux for 2 hours longer. The cooled mixture was diluted with diethyl ether and treated with water to dissolve the suspended salts. The ether was separated and the aqueous layer was extracted with diethyl ether. The combined ether extracts were washed, dried, concentrated and Claisen distilled to give 36.5 g of 2,2,4-trimethyl-3-cyclohexen-1-one, b.p. 60°C (10 mm, 1330 Pa).

To a stirred solution of 27.6 g of this product in 250 ml of ethanol was added portionwise 7.6 g of sodium borohydride. A cooling bath was used to hold the temperature at 25—30°C. After 2 hours the mixture was poured into water and extracted three times with methylene chloride. The combined methylene chloride extracts were washed, dried, concentrated and distilled to give 27.7 g of the desired product.

Example 8

1,2,2,4-Tetramethyl-3-cyclohexen-1-ol

To a stirred solution of 80 ml of 2.9 M methyl magnesium chloride (in tetrahydrofuran) in 200 ml of dry tetrahydrofuran was added dropwise at 25—30°C in a solution of 27.6 g of 2,2,4-trimethyl-3-cyclohexen-1-one in 30 ml of tetrahydrofuran. After 1 hour longer at 25°C and 1 hour at 45—50°C, the mixture was cooled and treated carefully with 50 ml of saturated ammonium sulphate. The mixture was extracted twice with diethyl ether and the combined ether extracts were dried, concentrated and distilled to give 12.5 g (*A*), b.p. 115—125°C (100 mm, 13290 Pa), which was mainly unchanged ketone starring material, 11.1 g (*B*), b.p. 125—115°C (100—50 mm, 13290—6645 Pa), which was the desired product of 81% purity. The final cut of 2.0 g (*C*), b.p. 115—120°C (50—20 mm, 6645—2260 Pa) was the desired product of 87% purity.

Example 9

1-Ethoxycarbonylmethyl-4-methyl-3-cyclohexen-1-ol

To a stirred mixture of 26.0 g of zinc dust, 2.0 g of iodine and 40 ml of benzene was added rapidly at 65—75°C a solution of 22.0 g of 4-methyl-3-cyclohexen-1-one and 62.6 g of ethyl bromoacetate in 400 ml of benzene. After 5 hours at reflux the mixture was cooled to below 10°C and treated dropwise with 300 ml of 10% acetic acid. After 15 minutes, the layers were separated and the aqueous layer was extracted twice with 150 ml of benzene. The combined organic layers were washed successively with water, sodium

bicarbonate solution and water. After drying and concentration, Claisen distillation gave 33.0 g of the desired product, b.p. 82—84°C (0.5 mm, 66 Pa).

Example 10

1,2,2,4,6,6-Hexamethyl-3-cyclohexen-1-ol

To a stirred solution of 27.6 g of 2,2,4-trimethyl-3-cyclohexen-1-one and 62.5 g of methyl iodide in 350 ml of tetrahydrofuran was added portionwise with cooling at 25—35°C 20.2 g of 50% sodium hydride. The reaction was completed by refluxing for an hour longer.

The cooled mixture was filtered and the filter cake was washed with tetrahydrofuran. The filtrate was concentrated to low volume and poured into water. Three extractions with methylene chloride, followed by washing, drying, concentration and Claisen-distillation, gave 25.6 g of 2,2,4,6,6-pentamethyl-3-cyclohexen-1-one. This was reacted with methyl magnesium bromide and reduced, as described in Example 7, to produce the desired product.

Examples 11 to 13

These examples describe the preparation of compounds of the general formula II.

Example 11

3,4-cis-Epoxy-1-isopropyl-4-methylcyclohexanol

To a solution of 30.8 g of (+)-terpinen-4-ol in 250 ml of toluene containing 1.0 g of vanadium (IV) bis(2,4-pentanedioate) oxide held at 45°C was added 22.0 g of 90% tert-butyl hydroperoxide. Cooling was used to maintain the reaction temperature at 45—50°C for several minutes. After two hours longer at the same temperature, the mixture was cooled, washed with 1 N sodium hydroxide, dried and Claisen distilled to give 30.6 g of the desired product, b.p. 75°C (2 mm, 266 Pa).

Example 12

cis-3,4-Epoxy-2,2,4-trimethylcyclohexanol

A solution of 27.7 g of the alcohol of Example 7 above in 250 ml of methylene chloride was treated with 1.0 g of vanadium (IV) bis(2,4-pentanedioate) oxide and 22.0 g of 90% tert-butylhydroperoxide. After stirring overnight at 25°C, the mixture was washed with 1 N sodium hydroxide, dried and Claisen distilled to give 23.7 g of the desired product, b.p. 58—61°C (1 mm, 133 Pa).

Example 13

3,4-cis-Epoxy-1-isopropyl-4-methylcyclohexanol

To a stirred, refluxing mixture of 15.4 g of terpinen-4-ol, 150 ml of propylene oxide and 0.3 g of vanadium (IV) bis(2,4-pentanedioate) oxide was added dropwise over 15 minutes 5.4 g of 70% hydrogen peroxide. After 45 minutes longer, the mixture was vacuum-concentrated at 60°C. The residue was shaken with a mixture of pentane and water and the dried pentane layer was vacuum concentrated to a residue of 11.2 g. This product was identified as 3,4-*cis*-epoxy-1-isopropyl-4-methylcyclohexanol by GLC and infrared.

Examples 14 to 17

These Examples describe the preparation of compounds of the general formula I.

Example 14

(±)-2-*exo*-Hydroxy-1-methyl-4-isopropyl-7-oxabicyclo[2,2,1]heptane

To a solution of 30.8 g of (±)-terpinen-4-ol and 0.8 g of vanadium (IV) bis(2,4-pentanedioate) oxide in 300 ml of methylene chloride was added 22.0 g of 90% tert-butyl hydroperoxide. The resulting reaction was held at reflux for 2 hours, after which 0.8 g of *p*-toluenesulphonic acid in 10 ml of glyme was added. The resulting reaction mixture was refluxed for an additional 1.5 hours, cooled and 0.8 g of anhydrous sodium acetate was added with stirring. After filtration, the filtrate was concentrated and Claisen distilled to give 28.4 g of the desired product, b.p. 80—95° (2 mm, 266 Pa).

Example 15

(−)-2-*exo*-Hydroxy-1-methyl-4-isopropyl-7-oxabicyclo[2,2,1]heptane

The procedure of Example 14 was repeated using (−)-terpinen-4-ol ($[\alpha]_D$ −28° ($CHCl_3$)). The distilled product was recrystallized from hexane to give the desired product having m.p. 83—85°C and $[\alpha]_D$ +0.4° ($CHCl_3$).

Example 16

(±)-2-*exo*-Hydroxy-1-methyl-4-(1-methyl-1-(-phenylsulphonyl)-ethyl)-7-oxabicyclo[2,2,1]heptane

To a stirred, refluxing solution of 13.1 g of (±)-4-methyl-1-(1-methyl-1-(phenylthio)ethyl)-3-cyclohexen-1-ol and 0.27 g of vanadium(IV) bis(2,4-pentanedioate) oxide in 130 ml of methylene chloride was added dropwise 10.0 g of 90% tert-butyl hydroperoxide over 10 minutes. The mixture was refluxed for one hour longer, and after cooling, washed, dried and vacuum concentrated at 50—55°C. To the resulting residue of about 18 g 5 ml of glyme containing 0.4 g of p-toluenesulphonic acid was added. The mixture was stirred

overnight at 5—25°C. Since an insoluble oil had formed, 100 ml of chloroform was added and the ether and pentane were removed by vacuum concentration. The residual chloroform solution was washed with potassium carbonate, dried and concentrated to a residue of 14.9 g. This residue was purified by dry column chromatography using a 30:220:500 mixture of tetrahydrofuran:ethyl acetate:hexane as eluent. This column was divided into 12 equal parts; fraction 10 gave 5 g of product. Recrystallization of fraction 10 from diethyl ether gave 3.0 g of the desired product, m.p. 108—110°C.

Example 17

2-Exo-hydroxy-1,3,3-trimethyl-7-oxabicyclo[2,2,1]heptane

In a method analogous to those described in Examples 14 to 16, the epoxy-alcohol of Example 12 above was used to prepare a product, 11.1 g, b.p. 55—77°C (1.0—0.1 mm; 133—13 Pa), which, on redistillation, gave 2.8 g of the desired product, b.p. 77—80°C (3.0 mm; 400 Pa).

Examples 18 to 26

By methods analogous to those described in Examples 14 to 17, further compounds of the general formula I were prepared. Details are given in Table I. All compound prepared were racemic mixtures.

TABLE I

| Example No. | In the general formula I $R^1$ | $R^2$ | $R^3$ | $R^4$ | $R^5$ | Boiling Point (°C(mmHg)) |
|---|---|---|---|---|---|---|
| 18 | $CH_3$ | $C_2H_5$ | H | H | H | 72—74(1.5) |
| 19 | $C_2H_5$ | $CH_3$ | H | H | H | 84—105(2) |
| 20 | $CH_3$ | $CH_3$ | H | H | H | 80—87(5) |
| 21 | n-$C_4H_9$ | $CH_3$ | H | H | H | 95—100(1.5) |
| 22 | $CH_3$ | n-$C_3H_7$ | H | H | H | 74—76(1) |
| 23 | phenyl | $CH_3$ | H | H | H | 112—118(0.15) |
| 24 | $C_2H_5$ | $C_2H_5$ | H | H | H | 65—78(1) |
| 25 | $CH_3$ | $CH_3$ | H | H | both $CH_3$ | 60—80(0.1) |
| 26 | $C_2H_5O\cdot CO\cdot CH_2$ | $CH_3$ | H | H | H | Not measured |

Examples 27 to 34

By methods analogous to those described in Examples 14 to 17, further compounds of the general formula I were prepared. However, rather than being isolated and characterized, they were converted into ethers *in situ* by reaction with benzyl chloride, and the resulting benzyl ethers, which fall within the scope of the subject-matter claimed in our co-pending application EP0081893, were characterized.

The general method was as described below:

To a solution of 1.7 g of (±)-2-*exo*-hydroxy-1-methyl-4-isopropyl-7-oxabicyclo[2,2,1]heptane in 15 ml of dimethylformamide was added at room temperature 0.5 g of 50% sodium hydride. The resulting mixture was stirred overnight at room temperature, heated for an hour at 50°C, cooled to room temperature, and after 1.5 g of benzyl chloride was added in one portion, stirred at room temperature for three hours, heated to 50°C for one hour, cooled, poured into 50 ml of water, and extracted with one 50 ml and two 25 ml portions of methylene chloride. The combined methylene chloride extracts were washed with 100 ml of water, dried and evaporated to give an orange oil. Claisen distillation yielded 1.5 g of (±)-2-*exo*-benzyloxy-1-methyl-4-isopropyl-7-oxabicyclo[2,2,1]heptane, b.p. 103°C at 0.08 mm; 11Pa).

Details of the compounds of the general formula I, and their ethers, are given in Table II. In all cases, the compounds prepared were racemic mixtures, and in all cases except Example 34, all of $R^3$, $R^4$ and $R^5$ in the general formula I were hydrogen atoms, and $R^2$ was a methyl group.

TABLE II

| Example No. | In the general formula I $R^1$ | After etherification; boiling point of benzyl ether (°C(mm Hg)) |
|---|---|---|
| 27 | 1-methyl-1-methylsulphonylethyl | not measured |
| 28 | 1-chloro-1-methylethyl | 120—122(0.15) |
| 29 | 1-cyano-1-methylethyl | 139—140(0.1) |
| 30 | 1-hydroxy-1-methylethyl | 114—115(0.1) |
| 31 | 1-methoxy-1-methylethyl | 110—115(0.1) |
| 32 | 1-ethoxy-1-methylethyl | 120—125(0.2) |
| 33 | 1-isopropoxy-1-methylethyl | 120—130 (0.2) |
| 34 | The compound: 2-*exo*-hydroxy-1,3,3 4,5,5-hexamethyl-7-oxabicyclo-[2,2,1]heptane | 110—115(0.1) |

**Claims for the Contracting States: BE CH DE FR GB IT LI LU NL SE**

1. A process for the preparation of a 2-*exo*-hydroxy-7-oxabicyclo[2,2,1]heptane compound of the general formula I characterized by cyclising, by treatment with an acid, a *cis* 3,4-epoxy cyclohexanol compound of the general formula II

(I) 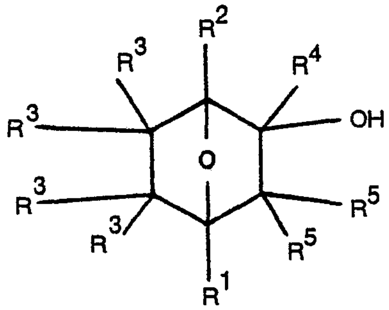

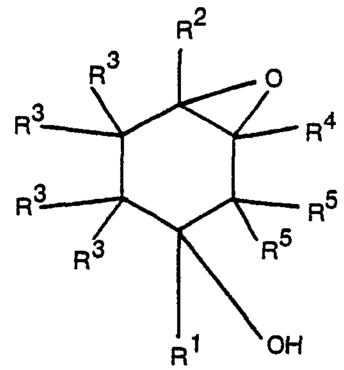 (II)

in which formulae

$R^1$ is a hydrogen atom, a $C_{1-10}$ alkyl group optionally substituted by up to 3 fluorine, chlorine and/or bromine atoms or by a hydroxy group, a cyano group, a $C_{1-4}$ alkoxy group, a $C_{1-6}$ alkylsulphonyl group, a $C_{6-10}$ arylsulphonyl group, a $C_{7-11}$ aralkylsulphonyl group, an azido group, a $C_{1-6}$ alkylcarbonyl group, a hydroxycarbonyl group, a phosphoryl group, a phosphoryloxy group, or an amine oxide, carbamoyl or thiocarbamoyl group in which the nitrogen atom is optionally substituted by 1 or 2 $C_{1-4}$ alkyl groups; an aryl or aralkyl group, each containing from 6 to 11 carbon atoms, and 1 to 4 carbon atoms in any alkyl portion, each optionally ring-substituted by one or more fluorine, chlorine and/or bromine atoms or by a $C_{1-2}$ alkyl or alkoxy group optionally substituted by one or more fluorine and/or chlorine atoms; or a group $CO_2R^6$ or $CON(R^6)_2$ in which $R^6$ is a hydrogen atom of a $C_{1-6}$ alkyl group;

$R^2$ is a hydrogen atom or a straight-chain $C_{1-6}$ alkyl group;

each $R^3$ is independently a hydrogen, chlorine or bromine atom, or a $C_{1-4}$ alkyl group optionally substituted by up to 3 fluorine, chlorine and/or bromine atoms; or two $R^3$'s when located on adjacent carbon atoms together form an epoxide ring or a carbon-carbon bond;

$R^4$ is a hydrogen atom or a $C_{1-4}$ alkyl group optionally substituted by up to 3 fluroine, chlorine and/or bromine atoms;

each $R^5$ is independently a hydrogen atom; a $C_{1-4}$ alkyl group optionally substituted by up to 3 fluorine chlorine and/or bromine atoms; a hydroxy group; or a $C_{1-4}$ alkoxy group.

2. A process according to claim 1, characterized in that a compound of formula II is used wherein $R^1$ is a hydrogen atom; A $C_{1-4}$ alkyl group optionally substituted by up to 3 fluorine, chlorine and/or bromine

atoms or by OH, CN, a $C_{1-4}$ alkoxy group, a $C_{1-6}$ alkylsulphonyl group, a phenylsulphonyl group, or a benzylsulphonyl group; or an aryl or aralkyl group each containing from 6 to 10 carbon atoms and 1 or 2 carbon atoms in any alkyl portion, optionally substituted by one or more fluorine, chlorine and/or bromine atoms or by a $C_{1-2}$ alkyl or alkoxy group each optionally substituted by one or more fluorine and/or chlorine atoms.

3. A process according to claim 1 or 2, characterized in that a compound of formula II is used wherein $R^1$ is a hydrogen atom or a $C_{1-3}$ alkyl group optionally substituted by chlorine.

4. A process according to any preceding claim, characterized in that a compound of formula II is used, wherein $R^2$ is a straight-chain $C_{1-4}$ alkyl group, each $R^3$ is a hydrogen, chlorine or bromine atom or a methyl or ethyl group, $R^4$ is a hydrogen atom, and $R^5$ is a hydrogen atom or a methyl or ethyl group.

5. A process according to any preceding claim, characterized in that a compound of formula II is used wherein $R^1$ is an isopropyl group, $R^2$ is a methyl group, $R^1$ and $R^2$ are both ethyl groups or $R^1$ is a 1-chloro-1-methylethyl group and $R^2$ is a methyl group.

6. A process according to any preceding claims, characterized in that the *cis* 3,4-epoxy cyclohexanol is treated with an acid having a $pK_A$ of 1 or less.

7. A process according to any preceding claims, characterized in that the acid is an organic sulphonic acid.

8. A process according to claim 7, characterized in that the acid is p-toluene sulphonic acid.

9. A process according to any preceding claim, characterized in that the amount of acid used is in the range of 0.02 to 0.04 mole of acid per mole of *cis*-epoxy cyclohexanol.

10. A process according to any preceding claim, 1—10 characterized in that the reaction is performed in the presence of an inert solvent.

11. A process according to any preceding claims, characterized in that the reaction is carried out at a temperature from 5—40°C.

12. A process according to any preceding claims, characterized in that the resulting compound of formula I is processed further without being previously recovered from the reaction mixture.

13. A process according to any preceding claims, characterized in that a compound of formula I is prepared which is subsequently converted into an ether derivative by reaction with a compound $WCQ_2$ L in which W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms, optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen; $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; aminocarbonyl, amino and carboxyl in each of which hydrogen may be replaced by $C_{1-4}$ alkyl; and $C_{1-3}$ alkyl which is itself optionally substituted by halogen, hydroxy, $C_{1-4}$ alkylthio, $C_{1-4}$ alkoxy, and amino; and both of Q are hydrogen atoms or fluorine atoms and L is a leaving group.

14. A process according to claim 13, characterized in that the leaving group L is selected from halogen atoms and organic sulphonyloxy groups.

15. A process according to any preceding claim, characterized in that as starting material a *cis* 3, 4-epoxy cyclohexanol is used prepared by epoxidation of the corresponding cyclohexenol with the aid of an oxidising agent.

16. A process according to claim 15, characterized in that the *cis* 3,4-epoxy cyclohexanol is prepared with the aid of an alkylhydroperoxide in the presence of a transition metal catalyst.

17. A process according to claim 15 or 16, characterized in that the *cis* 3,4-epoxycyclohexanol is converted to the 2-*exo*-hydroxy-7-oxabicyclo[2,2,1]heptane without previous isolation.

18. A compound of the general formula I

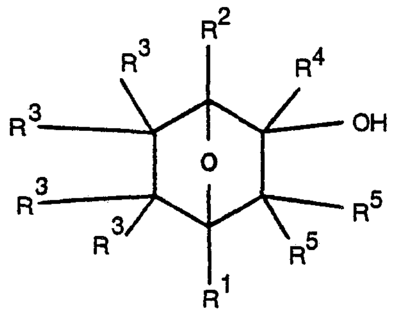

(I)

wherein $R^1$, $R^2$, $R^3$, $R^4$ and $R^5$ have the meanings as defined in any of claims 1 to 5, with the proviso that when $R^2$ represents a methyl group and all of $R^3$, $R^4$ and $R^5$ represent hydrogen atoms, $R^1$ is other than an isopropyl group.

19. A compound of the general formula II

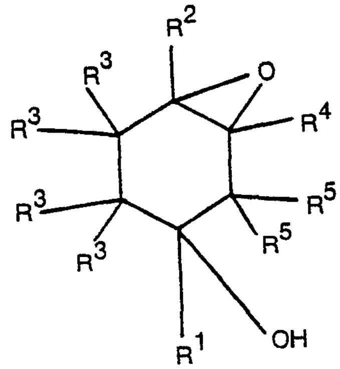

(II)

wherein $R^1$, $R^2$, $R^3$, $R^4$ and $R^5$ have the meanings as defined in any of claims 1 to 5, with the proviso that when $R^2$ represents a methyl group and all of $R^3$, $R^4$ and $R^5$ represent hydrogen atoms, $R^1$ is other than an isopropyl group.

20. A compound as claimed in claim 18 whenever prepared according to a process as claimed in any of claims 1—11.

**Claims for the Contracting State: AT**

1. A process for the preparation of a 2-*exo*-hydroxy-7-oxabicyclo[2,2,1]heptane compound of the general formula I, characterized by cyclising, by treatment with an acid, a *cis* 3,4-epoxy cyclohexanol compound of the general formula II

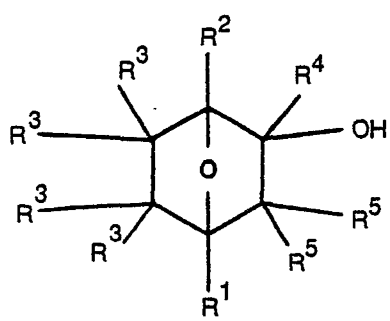

(I)

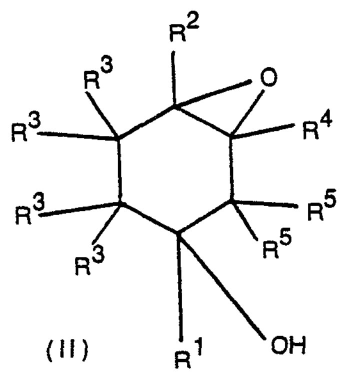

(II)

in which formulae

$R^1$ is a hydrogen atom, a $C_{1-10}$ alkyl group optionally substituted by up to 3 fluorine, chlorine and/or bromine atoms or by a hydroxy group, a cyano group, a $C_{1-4}$ alkoxy group, a $C_{1-6}$ alkylsulphonyl group, a $C_{6-10}$ arylsulphonyl group, a $C_{7-11}$ aralkylsulphonyl group, an azido group, a $C_{1-6}$ alkoxycarbonyl group, a hydroxycarbonyl group, a phosphoryl group, a phosphoryloxy group, or an amine oxide, carbamoyl or thiocarbamoyl group in which the nitrogen atom is optionally substituted by 1 or 2 $C_{1-4}$ alkyl groups; an aryl or aralkyl group, each containing from 6 to 11 carbon atoms, and 1 to 4 carbon atoms in any alkyl portion, each optionally ring-substituted by one or more fluorine, chlorine and/or bromine atoms or by a $C_{1-2}$ alkyl or alkoxy group optionally substituted by one or more fluorine and/or chlorine atoms; or a group $CO_2R^6$ or $CON(R^6)_2$ in which $R^6$ is a hydrogen atom or a $C_{1-6}$ alkyl group;

$R^2$ is a hydrogen atom or a straight-chain $C_{1-6}$ alkyl group;

each $R^3$ is independently a hydrogen, chlorine or bromine atom, or a $C_{1-4}$ alkyl group optionally substituted by up to 3 fluorine, chlorine and/or bromine atoms; or two $R^3$'s when located on adjacent carbon atoms together form an epoxide ring or a carbon-carbon bond;

$R^4$ is a hydrogen atom or a $C_{1-4}$ alkyl group optionally substituted by up to 3 fluorine, chlorine and/or bromine atoms;

each $R^5$ is independently a hydrogen atom; a $C_{1-4}$ alkyl group optionally substituted by up to 3 fluorine chlorine and/or bromine atoms; a hydroxy group; or a $C_{1-4}$ alkoxy group.

2. A process according to claim 1, characterized in that a compound of formula II is used wherein $R^1$ is a hydrogen atom; a $C_{1-4}$ alkyl group optionally substituted by up to 3 fluorine, chlorine and/or bromine atoms or by OH, CN, a $C_{1-4}$ alkoxy group, a $C_{1-6}$ alkylsulphonyl group, a phenylsulphonyl group, or a benzylsulphonyl group; or an aryl or aralkyl group each containing from 6 to 10 carbon atoms and 1 or 2 carbon atoms in any alkyl portion, optionally substituted by one or more fluorine, chlorine and/or bromine

atoms or by a $C_{1-2}$ alkyl or alkoxy group each optionally substituted by one or more fluorine and/or chlorine atoms.

3. A process according to claim 1 or 2, characterized in that a compound of formula II is used wherein $R^1$ is a hydrogen atom or a $C_{1-3}$ alkyl group optionally substituted by chlorine.

4. A process according to any preceding claim, characterized in that a compound of formula II is used, wherein $R^2$ is a straight-chain $C_{1-4}$ alkyl group, each $R^3$ is a hydrogen, chlorine or bromine atom or a methyl or ethyl group, $R^4$ is a hydrogen atom, and $R^5$ is a hydrogen atom or a methyl or ethyl group.

5. A process according to any preceding claim, characterized in that a compound of formula II is used wherein $R^1$ is an isopropyl group, $R^2$ is a methyl group, $R^1$ and $R^2$ are both ethyl groups or $R^1$ is a 1-chloro-1-methylethyl group and $R^2$ is a methyl group.

6. A process according to any preceding claims, characterized in that the *cis* 3,4-epoxy cyclohexanol is treated with an acid having a $pK_A$ of 1 or less.

7. A process according to any preceding claims, characterized in that the acid is an organic sulphonic acid.

8. A process according to claim 7, characterized in that the acid is p-toluene sulphonic acid.

9. A process according to any preceding claim, characterized in that the amount of acid used is in the range of 0.02 to 0.04 mole of acid per mole of *cis*-epoxy cyclohexanol.

10. A process according to any preceding claim, 1—10 characterized in that the reaction is performed in the presence of an inert solvent.

11. A process according to any preceding claims, characterized in that the reaction is carried out at a temperature from 5—40°C.

12. A process according to any preceding claims, characterized in that the resulting compound of formula I is processed further without being previously recovered from the reaction mixture.

13. A process according to any preceding claims, characterized in that a compound of formula I is prepared which is subsequently converted into an ether derivative by reaction with a compound $WCQ_2$ L in which W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms, optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen; $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; aminocarbonyl, amino and carboxyl in each of which hydrogen may be replaced by $C_{1-4}$ alkyl; and $C_{1-3}$ alkyl which is itself optionally substituted by halogen, hydroxy, $C_{1-4}$ alkylthio, $C_{1-4}$ alkoxy, and amino; and both of Q are hydrogen atoms or fluorine atoms and L is a leaving group.

14. A process according to claim 13, characterized in that the leaving group L is selected from halogen atoms and organic sulphonyloxy groups.

15. A process according to any preceding claim, characterized in that as starting material a *cis* 3, 4-epoxy cyclohexanol is used which has been prepared by epoxidation of the corresponding cyclohexenol with the aid of an oxidising agent.

16. A process according to claim 15, characterized in that the *cis* 3,4-epoxy cyclohexanol is prepared with the aid of an alkylhydroperoxide in the presence of a transition metal catalyst.

17. A process according to claim 15 or 16, characterized in that the *cis* 3,4-epoxycyclohexanol is converted to the 2-*exo*-hydroxy-7-oxabicyclo[2,2,1]heptane without previous isolation.

**Patentansprüche für die Vertragsstaaten: BE CH DE FR GB IT LI LU NL SE**

1. Verfahren zur Herstellung einer 2-exo-Hydroxy-7-oxabicyclo-[2,2,1]-heptan-Verbindung der allgemeinen Formel I, dadurch gekennzeichnet, daß eine cis-3,4-Epoxycyclohexanol-Verbindung der allgemeinen Formel II durch Behandlung mit einer Säure cyclisiert wird:

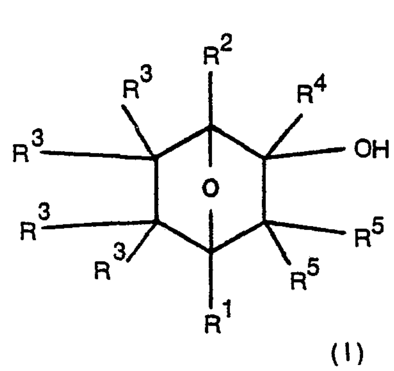

(I)

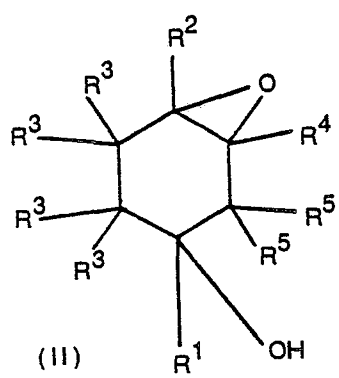

(II)

in welchem Formeln

$R^1$ ein Wasserstoffatom, eine $C_{1-10}$-Alkylgruppe, die gegebenenfalls durch bis zu 3 Fluor-, Chlor- und/oder Bromatome oder eine Hydroxygruppe, eine Cyanogruppe, eine $C_{1-4}$-Alkoxygruppe, eine $C_{1-6}$-Alkylsulfonylgruppe, eine $C_{6-10}$-Arylsulfonylgruppe, eine $C_{7-11}$-Aralkylsulfonylgruppe, eine Azidogruppe, eine $C_{1-6}$-Alkoxycarbonylgruppe, eine Hydroxycarbonylgruppe, eine Phosphorylgruppe, eine Phosphoryloxygruppe oder eine Aminoxid-, Carbamoyl- oder Thiocarbamoylgruppe substituiert ist, worin das Stickstoffatom gegebenenfalls durch eine oder zwei $C_{1-4}$-Alkylgruppen substituiert ist; eine Aryl- oder Aralkylgruppe, jeweils enthaltend von 6 bis 11 Kohlenstoffatome und 1 bis 4 Kohlenstoffatome in einem etwa vorliegenden Alkylteil, jeweils gegebenenfalls im Ring durch ein oder mehrere Fluor-, Chlor- und/oder Bromatome oder durch eine $C_{1-2}$-Alkyl- oder Alkoxygruppe substituiert, die im gegebenen Falle durch ein oder mehrere Fluor- und/oder Chloratome substituiert sind; oder eine Gruppe $CO_2R^6$ oder $CON(R^6)_2$ darstellt, worin $R^6$ ein Wasserstoffatom oder eine $C_{1-6}$-Alkylgruppe bedeutet;

$R^2$ ein Wasserstoffatom oder eine geradkettige $C_{1-6}$-Alkylgruppe bedeutet;

jeder Rest $R^3$ unabhängig voneinander ein Wasserstoff-, Chlor- oder Bromatom oder eine $C_{1-4}$-Alkylgruppe bedeutet, die gegebenenfalls durch bis zu drei Fluor-, Chlor- und/oder Bromatome substituiert ist; oder zwei Reste $R^3$, wenn sie an benachbarten Kohlenstoffatomen angeordnet, sind, zusammen einen Epoxidring oder eine Kohlenstoff-Kohlenstoffbindung ausbilden;

$R^4$ ein Wasserstoffatom oder eine $C_{1-4}$-Alkylgruppe bedeutet, die gegebenenfalls durch bis zu drei Fluor-, Chlor- und/oder Bromatome substituiert ist; und jeder Rest

$R^5$ unabhängig voneinander ein Wasserstoffatom; eine $C_{1-4}$-Alkylgruppe, die gegebenenfalls durch bis zu 3 Fluor-, Chlor- und/oder Bromatome substituiert ist; eine Hydroxygruppe; oder eine $C_{1-4}$-Alkoxygruppe darstellt.

2. Verfahren nach Anspruch 1, dadurch gekennzeichnet, daß eine Verbindung der Formel II eingesetzt wird, worin $R^1$ ein Wasserstoffatom; eine $C_{1-4}$-Alkylgruppe, die gegebenenfalls durch bis zu 3 Fluor-, Chlor- und/oder Bromatome oder durch OH, CN, eine $C_{1-4}$-Alkoxygruppe, eine $C_{1-6}$-Alkylsulfonylgruppe, eine Phenylsulfonylgruppe oder eine Benzylsulfonylgruppe substituiert ist; doer eine Aryl- oder Aralkylgruppe bedeutet, die jeweils 6 bis 10 Kohlenstoffatome und 1 oder 2 Kohlenstoffatome in einem etwa vorliegenden Alkylteil enthalten und gegebenenfalls durch ein oder mehrere Fluor-, Chlor- und/oder Bromatome oder durch eine $C_{1-2}$-Alyl- oder Alkoxygruppe substituiert sind, welche gegebenenfalls jeweils durch 1 oder mehrere Fluor- und/oder Chloratome substituiert sind.

3. Verfahren nach Anspruch 1 oder 2, dadurch gekennzeichnet, daß eine Verbindung der Formel II eingesetzt wird, worin $R^1$ ein Wasserstoffatom oder eine gegebenenfalls durch Chlor substituierte $C_{1-3}$-Alkylgruppe bedeutet.

4. Verfahren nach einem der vorstehenden Ansprüche, dadurch gekennzeichnet, daß eine Verbindung der Formel II eingesetzt wird, worin $R^2$ eine geradkettige $C_{1-4}$-Alkylgruppe bedeutet, jeder Rest $R^3$ ein Wasserstoff-, Chlor- oder Bromatom oder eine Methyl- oder Ethylgruppe darstellt, $R^4$ ein Wasserstoffatome ist und $R^5$ ein Wasserstoffatom oder eine Methyl- oder Ethylgruppe bedeutet.

5. Verfahren nach einem der vorstehenden Ansprüche, dadurch gekennzeichnet, daß eine Verbindung der Formel II eingesetzt wird, worin $R^1$ eine Isopropylgruppe bedeutet, $R^2$ eine Methylgruppe darstellt, $R^1$ und $R^2$ beide Ethylgruppen sind oder $R^1$ eine 1-Chlor-1-methylethylgruppe darstellt und $R^2$ eine Methylgruppe ist.

6. Verfahren nach einem der vorstehenden Ansprüche, dadurch gekennzeichnet, daß das cis-3,4-Epoxycyclohexanol mit einer Säure mit einem $pK_A$-Wert von 1 oder darunter behandelt wird.

7. Verfahren nach einem der vorstehenden Ansprüche, dadurch gekennzeichnet, daß die Säure eine organische Sulfonsäure ist.

8. Verfahren nach Anspruch 7, dadurch gekennzeichnet, daß die Säure p-Toluolsulfonsäure ist.

9. Verfahren nach einem der vorstehenden Ansprüche, dadurch gekennzeichnet, daß die Menge an eingesetzter Säure im Bereich von 0,02 bis 0,04 Mol Säure je Mol cis-Epoxycyclohexanol liegt.

10. Verfahren nach einem der vorstehenden Ansprüche, dadurch gekennzeichnet, daß die Umsetzung in Gegenwart eines inerten Lösungsmittels ausgeführt wird.

11. Verfahren nach einem der vorstehenden Ansprüche, dadurch gekennzeichnet, daß die Umsetzung bei einer Temperatur von 5 bis 40°C ausgeführt wird.

12. Verfahren nach einem der vorstehenden Ansprüche, dadurch gekennzeichnet, daß die erhaltene Verbindung der Formel I ohne vorangehende Gewinnung aus dem Reaktionsgemisch weiter verarbeitet wird.

13. Verfahren nach einem der vorstehenden Ansprüche, dadurch gekennzeichnet, daß eine Verbindung der Formel I hergestellt wird, die anschließend durch Umsetzung mit einer Verbindung $WCQ_2L$ in ein Etherderivat übergeführt wird, in welcher Formel W eine Cyanogruppe; eine $C_{2-4}$-Alkenyl- oder -Alkinylgruppe; eine Pyrimidinyl-, Pyrazinyl-, Pyridazinyl-, Pyridyl-, Furyl-, Naphthyl-, Imidazolyl-, Triazolyl-, Thiadiazolyl-, 2-Chinolinyl-, 1-Isochinolinyl-, Pyrrolyl-, N-Methylimidazolyl-, N-Methylpyrazolyl-, Isoxazolyl-, Oxazolyl-, Isothiazolyl-, Thiazolyl-, Thienyl- oder 5-Methyl-2-furyl-Gruppe; oder eine Cyclohexenylgruppe, oder eine Cycloalkylgruppe mit bis zu 6 Kohlenstoffatomen, gegebenenfalls substituiert durch eine $C_{1-3}$-Alkylgruppe; oder eine $C_{3-10}$-sec.Alkylgruppe oder eine Phenylgruppe bedeutet, die unsubstituiert oder durch einen oder mehrere der folgenden Reste substituiert ist: Hydroxy, Cyano, Nitro, Halogen; $C_{1-3}$-Alkoxy, Halogenalkoxy, Alkylthio, Halogenalkylthio, Alkylsulfinyl, halogenalkylsulfinyl oder

Alkylsulfonyl; Alkenyl oder Alkinyl bis zu 4 Kohlenstoffatomen; Phenyl, Phenoxy, Benzyl oder Benzyloxy; Aminocarbonyl, Amino und Carboxyl, in welchem jeweils Wasserstoff durch $C_{1-4}$-Alkyl ersetzt sein kann; und $C_{1-3}$-Alkyl, das seinerseits gegebenenfalls durch Halogen, Hydroxy, $C_{1-4}$-Alkylthio, $C_{1-4}$-Alkoxy und Amino substituiert ist; und beide Reste Q Wasserstoffatome oder Fluoratome bedeuten und L eine Leaving-Gruppe darstellt.

14. Verfahren nach Anspruch 13, dadurch gekennzeichnet, daß die Leaving-Gruppe unter Halogenatomen und organischen Sulfonyloxygruppen ausgewählt wird.

15. Verfahren nach einem der vorstehenden Ansprüche, dadurch gekennzeichnet, daß als Ausgangsmaterial ein cis-3,4-Epoxycyclohexanol eingesetzt wird, das durch Epoxidieren des entsprechenden Cyclohexanols mit Hilfe eines Oxidationsmittels hergestellt worden ist.

16. Verfahren nach Anspruch 15, dadurch gekennzeichnet, daß das cis-3,4-Epoxycyclohexanol mit Hilfe eines Alkylhydroperoxids in Anwesenheit eines Übergangsmetallkatalysators hergestellt wird.

17. Verfahren nach Anspruch 15 oder 16, dadurch gekennzeichnet, daß das cis-3,4-Epoxycyclohexanol ohne vorangehende Isolierung in das 2-exo-Hydroxy-7-oxabicyclo-[2,2,1]-heptan übergeführt wird.

18. Eine Verbindung der allgemeinen Formel I

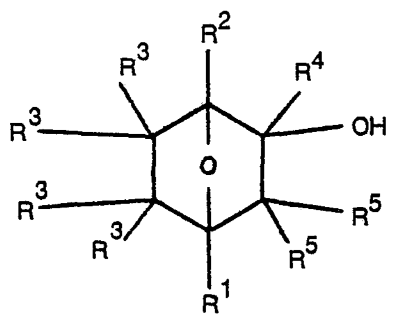

(I)

worin $R^1$, $R^2$, $R^3$, $R^4$ und $R^5$ die Bedeutungen, wie in einem der Ansprüche 1 bis 5 definiert, mit der Maßgabe aufweisen, daß dann, wenn $R^2$ eine Methylgruppe darstellt und alle Reste $R^3$, $R^4$ und $R^5$ Wasserstoffatome bedeuten, $R^1$ eine andere Bedeutung als die einer Isopropylgruppe aufweist.

19. Eine Verbindung der allgemeinen Formel II

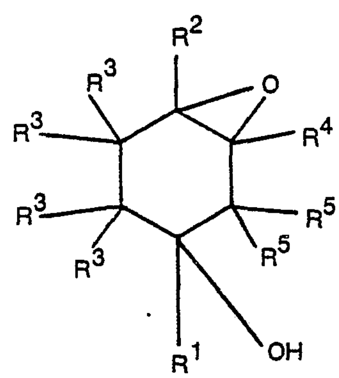

(II)

worin $R^1$, $R^2$, $R^3$, $R^4$ und $R^5$ die Bedeutungen, wie in einem der Ansprüche 1 bis 5 definiert, mit der Maßgabe aufweisen, daß dann, wenn $R^2$ eine Methylgruppe darstellt und alle Reste $R^3$, $R^4$ und $R^5$ Wasserstoffatome bedeuten, $R^1$ eine andere Bedeutung als die einer Isopropylgruppe aufweist.

20. Eine Verbindung, wie in Anspruch 18 beansprucht, wann immer sie nach einem Verfahren, wie in einem der Ansprüche 1 bis 11 beansprucht, hergestellt ist.

**Patentansprüche für den Vertragsstaat: AT**

1. Verfahren zur Herstellung einer 2-exo-Hydroxy-7-oxabicyclo-[2,2,1]-heptan-Verbindung der allgemeinen Formel I, dadurch gekennzeichnet, daß eine cis-3,4-Epoxycyclohexanol-Verbindung der allgemeinen Formel II durch Behandlung mit einer Säure cyclisiert wird:

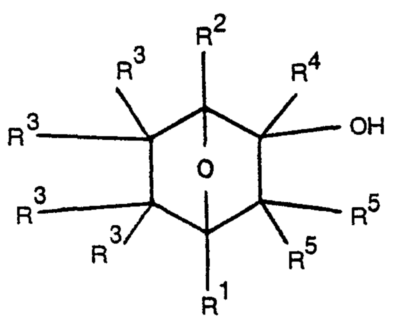

(I)

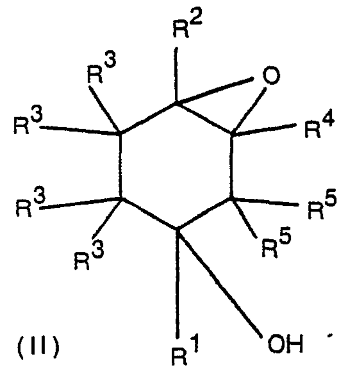

(II)

in welchem Formeln

$R^1$ ein Wasserstoffatom, eine $C_{1-10}$-Alkylgruppe, die gegebenenfalls durch bis zu 3 Fluor-, Chlor- und/oder Bromatome oder eine Hydroxygruppe, eine Cyanogruppe, eine $C_{1-4}$-Alkoxygruppe, eine $C_{1-6}$-Alkylsulfonylgruppe, eine $C_{6-10}$-Arylsulfonylgruppe, eine $C_{7-11}$-Aralkylsulfonylgruppe, eine Azidogruppe, eine $C_{1-6}$-Alkoxycarbonylgruppe, eine Hydroxycarbonylgruppe, eine Phosphorylgruppe, eine Phosphoryloxygruppe oder eine Amidoxid-, Carbamoyl- oder Thiocarbamoylgruppe substituiert ist, worin das Stickstoffatom gegebenenfalls durch eine oder zwei $C_{1-4}$-Alkylgruppen substituiert ist; eine Aryl- oder Aralkylgruppe, jeweils enthaltend von 6 bis 11 Kohlenstoffatome und 1 bis 4 Kohlenstoffatome in einem etwa vorliegenden Alkylteil, jeweils gegebenenfalls im Ring durch ein oder mehrere Fluor-, Chlor- und/oder Bromatome oder durch eine $C_{1-2}$-Alkyl- oder Alkoxygruppe substituiert, die im gegebenen Falle durch ein oder mehrere Fluor- und/oder Chloratome substituiert sind; oder eine Gruppe $CO_2R^6$ oder $CON(R^6)_2$ darstellt, worin $R^6$ ein Wasserstoffatom oder eine $C_{1-6}$-Alkylgruppe bedeutet;

$R^2$ ein Wasserstoffatom oder eine geradkettige $C_{1-6}$-Alkylgruppe bedeutet;

jeder Rest $R^3$ unabhängig voneinander ein Wasserstoff-, Chlor- oder Bromatom oder eine $C_{1-4}$-Alkylgruppe bedeutet, die gegebenenfalls durch bis zu drei Fluor-, Chlor- und/oder Bromatome substituiert ist; oder zwei Reste $R^3$, wenn sie an benachbarten Kohlenstoffatomen angeordnet sind, zusammen einen Epoxidring oder eine Kohlenstoff-Kohlenstoffbindung ausbilden;

$R^4$ ein Wasserstoffatom oder eine $C_{1-4}$-Alkylgruppe bedeutet, die gegebenenfalls durch bis zu drei Fluor-, Chlor- und/oder Bromatome substituiert ist; und jeder Rest

$R^5$ unabhängig voneinander ein Wasserstoffatom; eine $C_{1-4}$-Alkylgruppe, die gegebenenfalls durch bis zu 3 Fluor-, Chlor- und/oder Bromatome substituiert ist; eine Hydroxygruppe; oder eine $C_{1-4}$-Alkoxygruppe darstellt.

2. Verfahren nach Anspruch 1, dadurch gekennzeichnet, daß eine Verbindung der Formel II eingesetzt wird, worin $R^1$ ein Wasserstoffatom; eine $C_{1-4}$-Alkylgruppe, die gegebenenfalls durch bis zu 3 Fluor-, Chlor- und/oder Bromatome oder durch OH, CN, eine $C_{1-4}$-Alkoxygruppe, eine $C_{1-6}$-Alkylsulfonylgruppe, eine Phenylsulfonylgruppe oder eine Benzylsulfonylgruppe substituiert ist; oder eine Aryl- oder Aralkylgruppe bedeutet, die jeweils 6 bis 10 Kohlenstoffatome und 1 oder 2 Kohlenstoffatome in einem etwa vorliegenden Alkylteil enthalten und gegebenenfalls durch ein oder mehrere Fluor-, Chlor- und/oder Bromatome oder durch eine $C_{1-2}$-Alkyl- oder Alkoxygruppe substituiert sind, welche gegebenenfalls jeweils durch 1 oder mehrere Fluor- und/oder Chloratome substituiert sind.

3. Verfahren nach Anspruch 1 oder 2, dadurch gekennzeichnet, daß eine Verbindung der Formel II eingesetzt wird, worin $R^1$ ein Wasserstoffatom oder eine gegebenenfalls durch Chlor substituierte $C_{1-3}$-Alkylgruppe bedeutet.

4. Verfahren nach einem der vorstehenden Ansprüche, dadurch gekennzeichnet, daß eine Verbindung der Formel II eingesetzt wird, worin $R^2$ eine geradkettige $C_{1-4}$-Alkylgruppe bedeutet, jeder Rest $R^3$ ein Wasserstoff-, Chlor- oder Bromatom oder eine Methyl- oder Ethylgruppe darstellt, $R^4$ ein Wasserstoffatom ist und $R^5$ ein Wasserstoffatom oder eine Methyl- oder Ethylgruppe bedeutet.

5. Verfahren nach einem der vorstehenden Ansprüche, dadurch gekennzeichnet, daß eine Verbindung der Formel II eingesetzt wird, worin $R^1$ eine Isopropylgruppe bedeutet, $R^2$ eine Methylgruppe darstellt, $R^1$ und $R^2$ beide Ethylgruppen sind oder $R^1$ eine 1-Chlor-1-methylethylgruppe darstellt und $R^2$ eine Methylgruppe ist.

6. Verfahren nach einem der vorstehenden Ansprüche, dadurch gekennzeichnet, daß das cis-3,4-Epoxycyclohexanol mit einer Säure mit einem $pK_A$-Wert von 1 oder darunter behandelt wird.

7. Verfahren nach einem der vorstehenden Ansprüche, dadurch gekennzeichnet, daß die Säure eine organische Sulfonsäure ist.

8. Verfahren nach Anspruch 7, dadurch gekennzeichnet, daß die Säure p-Toluolsulfonsäure ist.

9. Verfahren nach einem der vorstehenden Ansprüche, dadurch gekennzeichnet, daß die Menge an eingesetzter Säure im Bereich von 0,02 bis 0,04 Mol Säure je Mol cis-Epoxycyclohexanol liegt.

10. Verfahren nach einem der vorstehenden Ansprüche, dadurch gekennzeichnet, daß die Umsetzung in Gegenwart eines inerten Lösungsmittels ausgeführt wird.

11. Verfahren nach einem der vorstehenden Ansprüche, dadurch gekennzeichnet, daß die Umsetzung bei einer Temperatur von 5 bis 40°C ausgeführt wird.

12. Verfahren nach einem der vorstehenden Ansprüche, dadurch gekennzeichnet, daß die erhaltene Verbindung der Formel I ohne vorangehende Gewinnung aus dem Reaktionsgemisch weiter verarbeitet wird.

13. Verfahren nach einem der vorstehenden Ansprüche, dadurch gekennzeichnet, daß eine Verbindung der Formel I hergestellt wird, die anschließend durch Umsetzung mit einer Verbindung $WCQ_2L$ in ein Etherderivat übergeführt wird, in welcher Formel W eine Cyanogruppe; eine $C_{2-4}$-Alkenyl- oder -Alkinylgruppe; eine Pyrimidinyl-, Pyrazinyl-, Pyridazinyl-, Pyridyl-, Furyl-, Naphthyl-, Imidazolyl-, Triazolyl-, Thiadiazolyl-, 2-Chinolinyl-, 1-Isochinolinyl-, Pyrrolyl-, N-Methylimidazolyl-, N-Methylpyrazolyl-, Isoxazolyl-, Oxazolyl-, Isothiazolyl-, Thiazolyl-, Thienyl- oder 5-Methyl-2-furyl-Gruppe; oder eine Cyclohexenylgruppe, oder eine Cycloalkylgruppe mit bis zu 6 Kohlenstoffatomen, gegebenenfalls substituiert durch eine $C_{1-3}$-Alkylgruppe; oder eine $C_{3-10}$-sec.Alkylgruppe oder eine Phenylgruppe bedeutet, die unsubstituiert oder durch einen oder mehrere der folgenden Reste substituiert ist: Hydroxy, Cyano, Nitro, Halogen; $C_{1-3}$-Alkoxy, Halogenalkoxy, Alkylthio, Halogenalkylthio, Alkylsulfinyl, Halogenalkylsulfinyl oder Alkylsulfonyl; Alkenyl oder Alkinyl bis zu 4 Kohlenstoffatomen; Phenyl, Phenoxy, Benzyl oder Benzyloxy; Aminocarbonyl, Amino und Carboxyl, in welchem jeweils Wasserstoff durch $C_{1-4}$-Alkyl ersetzt sein kann; und $C_{1-3}$-Alkyl, das seinerseits gegebenenfalls durch Halogen, Hydroxy, $C_{1-4}$-Alkylthio, $C_{1-4}$-Alkoxy und Amino substituiert ist; und beide Reste Q Wasserstoffatome oder Fluoratome bedeuten und L eine Leaving-Gruppe darstellt.

14. Verfahren nach Anspruch 13, dadurch gekennzeichnet, daß die Leaving-Gruppe unter Halogenatomen und organischen Sulfonyloxygruppen ausgewählt wird.

15. Verfahren nach einem der vorstehenden Ansprüche, dadurch gekennzeichnet, daß als Ausgangsmaterial ein cis-3,4-Epoxycyclohexanol eingesetzt wird, das durch Epoxidieren des entsprechenden Cyclohexanols mit Hilfe eines Oxidationsmittels hergestellt worden ist.

16. Verfahren nach Anspruch 15, dadurch gekennzeichnet, daß das cis-3,4-Epoxycyclohexanol mit Hilfe eines Alkylhydroperoxids in Anwesenheit eines Übergangsmetallkatalysators hergestellt wird.

17. Verfahren nach Anspruch 15 oder 16, dadurch gekennzeichnet, daß das cis-3,4-Epoxycyclohexanol ohne vorangehende Isolierung in das 2-exo-Hydroxy-7-oxabicyclo-[2,2,1]-heptan übergeführt wird.

**Revendications pour les Etats contractants: BE CH DE FR GB IT LI LU NL SE**

1. Procédé de préparation d'un composé 2-*exo*-hydroxy-7-oxabicyclo/2,2,1/heptane de formule générale I caractérisé par la cyclisation, par traitement avec un acide, d'un isomère *cis* d'un composé de 3,4-époxy-cyclohexanol de formule générale II

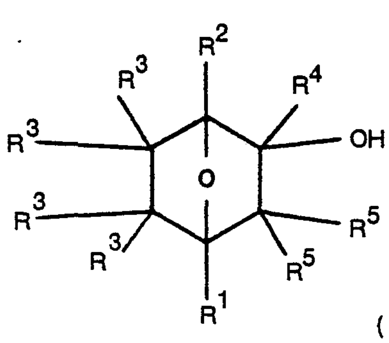
(I)

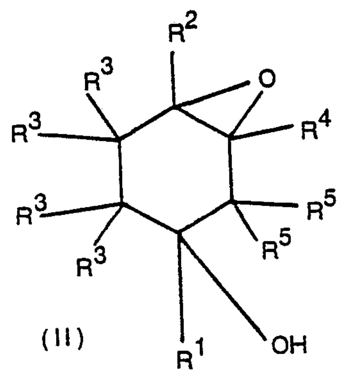
(II)

formules dans lesquelles

R1 est un atome d'hydrogène, un groupe alcoyle en C1 à C10 éventuellement substitué par jusqu'à 3 atomes de fluor, de chlore et/ou de brome ou par un groupe hydroxy, un groupe cyano, un groupe alcoxy en C1 à C4, un groupe alcoylsulfonyle en C1 à C6, un groupe arylsulfonyle en C6 à C10, un groupe aralcoylsulfonyl en C7 à C11, un groupe azido, un groupe alcoxycarbonyle en C1 à C6, un groupe hydroxycarbonyle, un groupe phosphoryle, un groupe phosphoryloxy, ou un groupe amine-oxyde, carbamoyle ou thiocarbamoyle dans lequel l'atome d'azote est éventuellement substitué par un ou deux groupes alcoyle en C1 à C4; un groupe aryle ou aralcoyle, contenant chacun de 6 à 11 atomes de carbone, et de 1 à 4 atomes de carbone dans toute fraction alcoyle, chacun éventuellement substitué dans son noyau

par un ou plusieurs atomes de fluor, de chlore et/ou de brome ou par un groupe alcoyle ou alcoxy en C1 à C2 éventuellement substitué par un ou plusieurs atomes de fluor et/ou de chlore; ou un groupe $CO_2$ R6 ou $CON(R6)_2$ où R6 est un atome d'hydrogène ou un groupe alcoyle en C1 à C6;

R2 est un atome d'hydrogène ou un groupe alcoyle en C1 à C6 à chaîne droite; chaque

R3 est indépendamment un atome d'hydrogène, de chlore ou de brome, ou un groupe alcoyle en C1 à C4 éventuellement substitué par jusqu'à 3 atomes de fluor, de chlore et/ou de brome; ou deux R3 lorsqu'ils sont situés sur des atomes de carbone adjacents forment ensemble un noyau époxyde ou une liaison carbone-carbone;

R4 est un atome d'hydrogène ou un groupe alcoyle en C1 à C4 éventuellement substitué par jusqu'à 3 atomes de fluor, de chlore et/ou de brome; chaque

R5 représente indépendamment un atome d'hydrogène; un groupe alcoyle en C1 à C4 éventuellement substitué par jusqu'à 3 atomes de fluor, de chlore et/ou de brome; un groupe hydroxy; ou un groupe alcoxy en C1 à C4.

2. Procédé selon la revendication 1, caractérisé en ce qu'on utilise un composé de formule II où R1 est un atome d'hydrogène; un groupe alcoyle en C1 à C4 éventuellement substitué par jusqu'à 3 atomes de fluor, de chlore et/ou de brome ou par OH, CN, un groupe alcoxy en C1 à C4, un groupe alcoylsulfonyle en C1 à C6, un groupe phénylsulfonyle ou un groupe benzylsulfonyle; ou un groupe aryle ou aralcoyle contenant chacun de 6 à 10 atomes de carbone et 1 ou 2 atomes de carbone dans toute fraction alcoyle, éventuellement substitué par un ou plusieurs atomes de fluor, de chlore et/ou de brome ou par un groupe alcoyle ou alcoxy chacun éventuellement substitué par un ou plusieurs atomes de fluor et/ou de chlore.

3. Procédé selon les revendications 1 et 2, caractérisé en ce qu'on utilise un composé de formule II où R1 est un atome d'hydrogène ou un groupe alcoyle en C1 à C3 éventuellement substitué par un chlore.

4. Procédé selon l'une quelconque des revendications précédentes, caractérisé en ce qu'on utilise un composé de formule II où R2 est un groupe alcoyle en C1 à C4 à chaîne droite, chaque R3 est un atome d'hydrogène, de chlore ou de brome ou un groupe méthyle ou éthyle, R4 est un atome d'hydrogène, et R5 est un atome d'hydrogène ou un groupe méthyle ou éthyle.

5. Procédé selon l'une quelconque des revendications précédentes, caractérisé en ce qu'on utilise un composé de formule II où R1 est un groupe isopropyle, R2 est un groupe méthyle, R1 et R2 sont tous deux des groupes éthyle ou R1 est un groupe 1-chloro-1-méthyléthyle et R2 est un groupe méthyle.

6. Procédé selon l'une quelconque des revendications précédentes, caractérisé en ce qu'on traite le *cis*-3,4-époxy-cyclohexanol avec un acide ayant un $pK_A$ égal ou inférieur à 1.

7. Procédé selon l'une quelconque des revendications précédentes, caractérisé en ce que l'acide est un acide sulfonique organique.

8. Procédé selon la revendication 7, caractérisé en ce que l'acide est l'acide p-toluènesulfonique.

9. Procédé selon l'une quelconque des revendications précédentes, caractérisé en ce que la quantité d'acide utilisé est dans un intervalle allant de 0,02 à 0,04 mole d'acide par mole de *cis*-époxy-cyclohexanol.

10. Procédé selon l'une quelconque des revendications précédentes, 1 à 10, caractérisé en ce que la réaction est conduite en présence d'un solvant inerte.

11. Procédé selon l'une quelconque des revendications précédentes, caractérisé en ce que la réaction est conduite à une température allant de 5 à 40°C.

12. Procédé selon l'une quelconque des revendications précédentes, caractérisé en ce qu'on traite plus avant le composé de formule I résultant sans le recueillir auparavant à partir du mélange réactionnel.

13. Procédé selon l'une quelconque des revendications précédentes, caractérisé en ce qu'on prépare un composé de formule I que l'on transforme ultérieurement en un dérivé éther par réaction avec un composé $WCQ_2$ — où W représente un groupe cyano; un groupe alcényle ou alcynyle en C2 à C4; un groupe pyrimidinyle, pyrazinyle, pyridazinyle, pyridyle, furyle, naphtyle, imidazolyle, triazolyle, thiadiazolyle, 2-quinolinyle, 1-isoquinolinyle, pyrrolyle, N-méthylimidazolyle, N-méthylpyrazolyle, isoxazolyle, oxazolyle, isothiazolyle, thiazolyle, thiényle ou 5-méthyl-2-furyle; ou un groupe cyclohexényle, ou un groupe cycloalcoyle ayant jusqu'à 6 atomes de carbone, éventuellement substitué par un groupe alcoyle en C1 à C3; ou un groupe alcoyle secondaire en C3 à C10; ou un groupe phényle qui est non substitué ou substitué par une ou plusieurs des fractions suivantes: hydroxy, cyano, nitro, halogène; alcoxy en C1 à C3, haloalcoxy, alcoylthio, haloalcoylthio, alcoylsulfinyle, haloalcoylsulfinyle ou alcoylsulfonyle; alcényle ou alcynyle ayant jusqu'à 4 atomes de carbone; phényle, phénoxy, benzyle ou benzyloxy; aminocarbonyle, amino et carboxyle dans chacun desquels l'hydrogène peut être remplacé par un alcoyle en C1 à C4; et alcoyle en C1 à C3 qui est lui-même éventuellement substitué par halogène, hydroxy, alcoylthio en C1 à C4, alcoxy en C1 à C4 et amino; et les deux Q représentent des atomes d'hydrogène ou des atomes de fluor, et L est un groupe sortant.

14. Procédé selon la revendication 13, caractérisé en ce que le groupe sortant L est choisi entre les atomes d'halogène et les groupes sulfonyloxy organiques.

15. Procédé selon l'une quelconque des revendications précédentes, caractérisé en ce qu'on utilise comme produit de départ un *cis*-3,4-époxycyclohexanol préparé par époxydation du cyclohexénol correspondant à l'aide d'un agent oxydant.

16. Procédé selon la revendication 15, caractérisé en ce qu'on prépare le *cis*-3,4-époxy-cyclohexanol à l'aide d'un alcoylhydroperoxyde en présence d'un catalyseur métal de transition.

17. Procédé selon les revendications 15 et 16, caractérisé en ce qu'on transforme le *cis*-3,4-

époxycyclohexanol en le 2-*exo*-hydroxy-7-oxabicyclo/2,2,1/heptane sans l'isoler antérieurement.

18. Composé de formule générale I

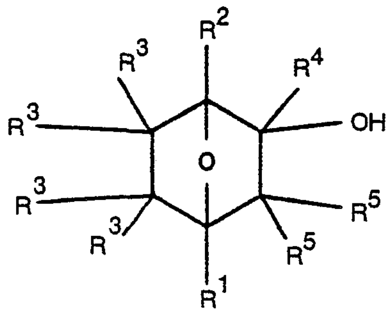

(I)

où R1, R2, R3, R4 et R5 ont les significations définies dans l'une quelconque des revendications 1 à 5, avec cette précision que lorsque R2 représente un groupe méthyle et tous les radicaux R3, R4 et R5 représentent des atomes d'hydrogène, R1 est différent d'un groupe isopropyle.

19. Composé de formule générale II

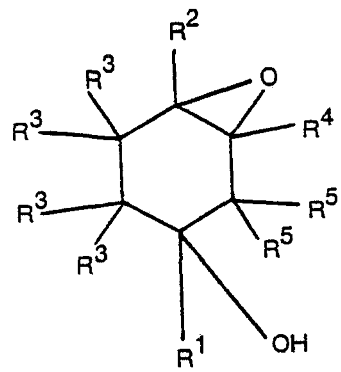

(II)

où R1, R2, R3, R4 et R5 ont les significations définies dans l'une quelconque des revendications 1 à 5, avec cette précision que lorsque R2 représente un groupe méthyle et tous les radicaux R3, R4 et R5 représentent des atomes d'hydrogène, R1 est différent d'un groupe isopropyle.

20. Composé tel que défini dans la revendication 18 lorsqu'on le prépare selon un procédé tel que revendiqué dans l'une quelconque des revendications 1 à 11.

**Revendications pour l'Etat contractant: AT**

1. Procédé de préparation d'un composé 2-*exo*-hydroxy-7-oxabicyclo/2,2,1/heptane de formule générale I caractérisé par la cyclisation, par traitement avec un acide, d'un isomère *cis* d'un composé de 3,4-époxy-cyclohexanol de formule générale II

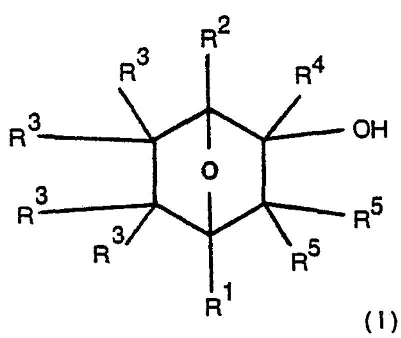

(I)

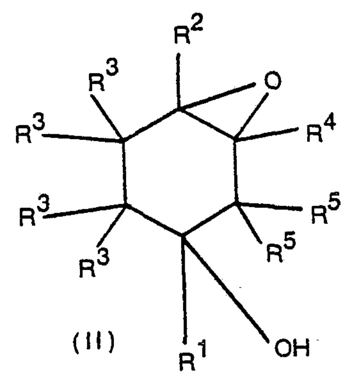

(II)

formules dans lesquelles

R1 est un atome d'hydrogène, un groupe alcoyle en C1 à C10 éventuellement substitué par jusqu'à 3 atomes de fluor, de chlore et/ou de brome ou par un groupe hydroxy, un groupe cyano, un groupe alcoxy en C1 à C4, un groupe alcoylsulfonyle en C1 à C6, un groupe arylsulfonyle en C6 à C10, un groupe aralcoylsulfonyle en C7 à C11, un groupe azido, un groupe alcoxycarbonyle en C1 à C6, un groupe

hydroxycarbonyle, un groupe phosphoryle, un groupe phosphoryloxy, ou un groupe amine-oxyde, carbamoyle ou thiocarbamoyle dans lequel l'atome d'azote est éventuellement substitué par un ou deux groupes alcoyle en C1 à C4; un groupe aryle ou aralcoyle, contenant chacun de 6 à 11 atomes de carbone, et de 1 à 4 atomes de carbone dans toute fraction alcoyle, chacun éventuellement substitué dans son noyau par un ou plusieurs atomes de fluor, de chlore et/ou de brome ou par un groupe alcoyle ou alcoxy en C1 à C2 éventuellement substitué par un ou plusieurs atomes de fluor et/ou de chlore; ou un groupe $CO_2$ R6 ou $CON(R6)_2$ où R6 est un atome d'hydrogène ou un groupe alcoyle en C1 à C6;

R2 est un atome d'hydrogène ou un groupe alcoyle en C1 à C6 à chaîne droite; chaque

R3 est indépendamment un atome d'hydrogène, de chlore ou de brome, ou un groupe alcoyle en C1 à C4 éventuellement substitué par jusqu'à 3 atomes de fluor, de chlore et/ou de brome; ou deux R3 lorsqu'ils sont situés sur des atomes de carbone adjacents forment ensemble un noyau époxyde ou une liaison carbone-carbone;

R4 est un atome d'hydrogène ou un groupe alcoyle en C1 à C4 éventuellement substitué par jusqu'à 3 atomes de fluor, de chlore e/ou de brome; chaque

R5 représente indépendamment un atome d'hydrogène; un groupe alcoyle en C1 à C4 éventuellement substitué par jusqu'à 3 atomes de fluor, de chlore et/ou de brome; un groupe hydroxy; ou un groupe alcoxy en C1 à C4.

2. Procédé selon la revendication 1, caractérisé en ce qu'on utilise un composé de formule II où R1 est un atome d'hydrogène; un groupe alcoyle en C1 à C4 éventuellement substitué par jusqu'à 3 atomes de fluor, de chlore et/ou de brome ou par OH, CN, un groupe alcoxy en C1 à C4, un groupe alcoylsulfonyle en C1 à C6, un groupe phénylsulfonyle ou un groupe benzylsulfonyle; ou un groupe aryle ou aralcoyle contenant chacun de 6 à 10 atomes de carbone et 1 ou 2 atomes de carbone dans toute fraction alcoyle, éventuellement substitué par un ou plusieurs atomes de fluor, de chlore et/ou de brome ou par un groupe alcoyle ou alcoxy chacun éventuellement substitué par un ou plusieurs atomes de fluor et/ou de chlore.

3. Procédé selon les revendications 1 et 2, caractérisé en ce qu'on utilise un composé de formule II où R1 est un atome d'hydrogène ou un groupe alcoyle en C1 à C3 éventuellement substitué par un chlore.

4. Procédé selon l'une quelconque des revendications précédentes, caractérisé en ce qu'on utilise un composé de formule II où R2 est un groupe alcoyle en C1 à C4 à chaîne droite, chaque R3 est un atome d'hydrogène, de chlore ou de brome ou un groupe méthyle ou éthyle, R4 est un atome d'hydrogène, et R5 est un atome d'hydrogène ou un groupe méthyle ou éthyle.

5. Procédé selon l'une quelconque des revendications précédentes, caractérisé en ce qu'on utilise un composé de formule II où R1 est un groupe isopropyle, R2 est un groupe méthyle, R1 et R2 sont tous deux des groupes éthyle ou R1 est un groupe 1-chloro-1-méthyléthyle et R2 est un groupe méthyle.

6. Procédé selon l'une quelconque des revendications précédentes, caractérisé en ce qu'on traite le *cis*-3,4-époxy-cyclohexanol avec un acide ayant un $pK_A$ égal ou inférieur à 1.

7. Procédé selon l'une quelconque des revendications précédentes, caractérisé en ce que l'acide est un acide sulfonique organique.

8. Procédé selon la revendication 7, caractérisé en ce que l'acide est l'acide p-toluènesulfonique.

9. Procédé selon l'une quelconque des revendications précédentes, caractérisé en ce que la quantité d'acide utilisé est dans un intervalle allant de 0,02 à 0,04 mole d'acide par mole de *cis*-époxy-cyclohexanol.

10. Procédé selon l'une quelconque des revendications précédentes, 1 à 10, caractérisé en ce que la réaction est conduite en présence d'un solvant inerte.

11. Procédé selon l'une quelconque des revendications précédentes, caractérisé en ce que la réaction est conduite à une température allant de 5 à 40°C.

12. Procédé selon l'une quelconque des revendications précédentes, caractérisé en ce qu'on traite plus avant le composé de formule I résultant sans le recueillir auparavant à partir du mélange réactionnel.

13. Procédé selon l'une quelconque des revendications précédentes, caractérisé en ce qu'on prépare un composé de formule I que l'on transforme ultérieurement en un dérivé éther par réaction avec un composé $WCQ_2$ — où W représente un groupe cyano; un groupe alcényle ou alcynyle en C2 à C4; un groupe pyrimidinyle, pyrazinyle, pyridazinyle, pyridyle, furyle, naphtyle, imidazolyle, triazolyle, thiadiazolyle, 2-quinolinyle, 1-isoquinolinyle, pyrrolyle, N-méthylimidazolyle, N-méthylpyrazolyle, isoxazolyle, oxazolyle, isothiazolyle, thiazolyle, thiényle ou 5-méthyl-2-furyle; ou un groupe cyclohexényle, ou un groupe cycloalcoyle ayant jusqu'à 6 atomes de carbone, éventuellement substitué par un groupe alcoyle en C1 à C3; ou un groupe alcoyle secondaire en C3 à C10; ou un groupe phényle qui est non substitué ou substitué par une ou plusieurs des fractions suivantes: hydroxy, cyano, nitro, halogène; alcoxy en C1 à C3, haloalcoxy, alcoylthio, haloalcoylthio, alcoylsulfinyle, haloalcoylsulfinyle ou alcoylsulfonyle; alcényle ou alcynyle ayant jusqu'à 4 atomes de carbone; phényle, phénoxy, benzyle ou benzyloxy; aminocarbonyle, amino et carboxyle dans chacun desquels l'hydrogène peut être remplacé par un alcoyle en C1 à C4; et alcoyle en C1 à C3 qui est lui-même éventuellement substitué par halogène, hydroxy, alcoylthio en C1 à C4, alcoxy en C1 à C4 et amino; et les deux Q représentent des atomes d'hydrogène ou des atomes de fluor, et L est un groupe sortant.

14. Procédé selon la revendication 13, caractérisé en ce que le groupe sortant L est choisi entre les atomes d'halogène et les groupes sulfonyloxy organiques.

15. Procédé selon l'une quelconque des revendications précédentes, caractérisé en ce qu'on utilise

comme produit de départ un *cis*-3,4-époxycyclohexanol préparé par époxydation du cyclohexénol correspondant à l'aide d'un agent oxydant.

16. Procédé selon la revendication 15, caractérisé en ce qu'on prépare le *cis*-3,4-époxy-cyclohexanol à l'aide d'un alcoylhydroperoxyde en présence d'un catalyseur métal de transition.

17. Procédé selon les revendications 15 et 16, caractérisé en ce qu'on transforme le *cis*-3,4-époxycyclohexanol en le 2-*exo*-hydroxy-7-oxabicyclo/2,2,1/heptane sans l'isoler antérieurement.